# EUROPÄISCHE PATENTSCHRIFT

(11) **EP 1 667 665 B1**
(45) Veröffentlichungstag und Bekanntmachung des Hinweises auf die Patenterteilung: **07.01.2009**
(21) Anmeldenummer: 04764591.6
(22) Anmeldetag: 28.08.2004
(51) Int. Cl.: A61K 9/50, A23L 1/302, A23L 1/303, A23L 1/304, A23L 1/305

(54) **KAPSELN ENTHALTEND WIRKSTOFFPELLETS MIT UNTERSCHIEDLICHEN FREISETZUNGSPROFILEN**
CAPSULES COMPRISING DRUG-LOADED PELLETS WITH DIFFERENT RELEASE PROFILES
CAPSULES COMPRENANT PELLETS AVEC DES AGENTS ACTIFS A LIBERATION DIFFERENTIEE

(30) Priorität: 03.09.2003 EP 03019577
(43) Veröffentlichungstag der Anmeldung: 14.06.2006
(73) Patentinhaber: Pharmaton S.A., 6934 Bioggio (CH)
(72) Erfinder: ANSCHÜTZ, Sergej, 55452 RÜMMELSHEIM (DE); SCHNEIDER, Roland, 65338 Schlangenbad (DE)
(74) Vertreter: Hammann, Heinz
(86) Internationale Anmeldenummer: PCT/EP2004/009617
(87) Internationale Veröffentlichungsnummer: WO 2005/023229

(56) Entgegenhaltungen:
- EP-A- 0 820 703
- WO-A-00/69420
- WO-A-98/19667
- FR-A- 2 705 233
- US-B1- 6 251 427

## Beschreibung

Die Erfindung betrifft Kapseln, enthaltend Wirkstoffpellets mit mindestens drei verschiedenen Wirkstoffen, wobei die Wirkstoffe ausgewählt sind aus der Gruppe der Nährstoffe, wie z.B. Vitamine, Mineralstoffe, Spurenelemente, ungesättigte Fettsäuren und Aminosäuren oder der Gruppe pflanzlicher Substanzen bzw. Extrakte.

### Hintergrund der Erfindung

Es ist eine seit langem bekannte Tatsache, dass Wirkstoffe dort freigesetzt werden sollten, wo sie vom Körper am effektivsten resorbiert werden können. Dies ist jedoch nicht ohne weiteres in die Praxis umzusetzen.

Bekannt sind Darreichungsformen, die derart beschichtet sind, dass sie die enthaltenen Arzneimittel über längere Zeit verteilt oder zeitverzögert freisetzen, wodurch eine so genannte Depot- oder Retardwirkung resultiert. Von diesen Arzneimittelformen sind solche mit einer kontrollierten oder mit einer modifizierten Wirkstoff-Freigabe zu unterscheiden. Die letzteren Formen kommen insbesondere dann zum Einsatz, wenn die Wirkstoffe gezielt an einen entlegenen Resorptionsort gelangen müssen. Die Versorgung mit einem Wirkstoff wird nämlich nicht durch die Zufuhr allein, sondern auch durch die Bioverfügbarkeit bestimmt. Für jeden Wirkstoff gibt es einen optimalen Freisetzungs- oder Resorptionsort. Dabei ist davon auszugehen, dass durch unterschiedliche pH-Werte, Temperaturen usw. während der Magen-Darm-Passage Verluste eintreten, die durch die vorliegende Erfindung vermieden werden können. Dies stellt insbesondere dann ein Problem dar, wenn unterschiedliche Wirkstoffe mit unterschiedlichen Resorptionsorten verabreicht werden.

Bei einem Präparat mit mehreren Wirkstoffbestandteilen besteht ferner die Gefahr, dass die vorhandenen Wirkstoffe sich gegenseitig beeinflussen, hierdurch bei der Resorption und Wirkung gehemmt oder sogar blockiert werden und somit ihr Wirkstoffspektrum herabgesetzt wird oder ihr Potential verloren geht. Auch Nahrungsmittelinhaltsstoffe können die Absorption von bestimmten Wirkstoffen herabsetzen. Beispielsweise setzt die Oxalsäure in Spinat oder die Phytinsäure in Vollkornprodukten die Calcium-Absorption herab. Andererseits kann die Absorption auch gesteigert werden. So stimuliert die gleichzeitige Gabe von Vitamin D die Resorption von Calcium im Dünndarm. Um die Wirkstoffe optimal zu nutzen, wäre daher eine komplexe und zeitlich genau kontrollierte Abfolge der Einnahme der verschiedenen Einzelsubstanzen notwendig, die aufgrund der Umständlichkeit und dem notwendigen Zeitaufwand nicht für den täglichen Gebrauch in Frage kommt.

Es gibt daher einige Vorschläge im Stand der Technik, die oben angesprochenen Probleme zu bewältigen:
Nach der WO 01/72286 A1 wird eine therapeutische Formulierung in Form von Perlen für die orale Verabreichung zur Verfügung gestellt, worin das Arzneimittel in kontrollierten Raten freigesetzt wird. Die Perlen sind aus drei Schichten aufgebaut, einem sphärisch extrudierten inneren Kern, der das verzögert freizusetzende Medikament enthält, einer äußeren Schicht, die ein sofort freizusetzendes Medikament enthält sowie einer zwischen beiden Schichten vorgesehenen Zwischenschicht, die zusätzlich die Freisetzung des Medikaments des inneren Kerns steuern kann. Demnach wird eine Darreichungsform vorgeschlagen, bei der in jedem einzelnen Pellet mehrere unterschiedliche Freisetzungsraten verwirklicht sind.

Gemäß der Lehre der US 3 939 259 werden therapeutische Zubereitungen beschrieben, bestehend aus Partikeln mit sich zersetzenden Beschichtungen von Zein und Schellack, die ein therapeutisch aktives Material enthalten, wobei die Freisetzung durch Variation der Schichtdicke gesteuert wird. Es können auch Vitamine zum Einsatz kommen.

Die Internationale Patentanmeldung WO 98/19667 schlägt eine "sustained release" Formulierung für den Onkologie-Wirkstoff DFMO vor.

Die Internationale Patentanmeldung WO 00/69420 beschreibt eine Darreichungsform für Vitamin C, bei dem das Vitamin C in Form von Mikrokapseln im Magendarmtrakt in 3 Freisetzungs-Pulsen freigesetzt wird.

Weiterhin beschäftigt sich das deutsche Gebrauchsmuster DE 202 02 984 U1 mit einer Mehrkammer-Kapsel zur zeit versetzt einsetzenden Auflösung elementarer Nährstoffe im Magen-Darm-Trakt, wobei die Kapsel aus mehreren ineinander oder aneinander gefügten Kammern besteht. Die einzelnen Kammern enthalten unterschiedliche Wirksubstanzen, wie Vitamine, Spurenelemente, Mineralstoffe und Aminosäuren. Es liegen demnach kompliziert ineinander geschachtelte Kapseln vor. Diese technische Lösung erscheint technisch sehr aufwendig, zudem aufgrund der komplexen Strukturelemente schlecht realisierbar und für den industriellen Einsatz nicht wirtschaftlich umsetzbar.

Die europäische Patentanmeldung EP 0 820 703 beschreibt Darreichungsformen, bei denen lipophile Wirkstoffe schnell (less than an hour) freigesetzt werden und hydrophile langsam (over eight hours) freigesetzt werden. Hinsichtlich des jeweiligen Freisetzungsortes gibt es dieser europäischen Patentanmeldung keine Aussage.

Die Bereitstellung eines Systems, welches die Formulierung und hinsichtlich des Ortes der Freisetzung gezielte Freigabe einer größeren Anzahl an verschiedenen Wirkstoffen ermöglicht, ohne auf die geschilderten komplizierten Strukturen zurückzugreifen, stellt daher nach wie vor ein ungelöstes Problem im Stand der Technik dar.

Der vorliegenden Erfindung liegt somit die Aufgabe zugrunde, eine vereinfachte Darreichungsform zur Verfügung zu stellen, die eine verbesserte Bioverfügbarkeit für Wirkstoffe liefert, insbesondere für Nährstoffe wie Vitamine, Mineralien, Spurenelemente, ungesättigte Fettsäuren, Aminosäuren oder pflanzliche Extrakte und Substanzen. Die Darreichungsform sollte Flexibilität hinsichtlich der Freisetzungsprofile bieten, so dass kontrolliert eine schnelle als auch verzögerte Freisetzung an den Resorptionsorten im Magen und in den entsprechenden Darmabschnitten gezielt möglich ist. Die Darreichungsform sollte ferner einfach und wirtschaftlich herstellbar sein.

### Detaillierte Beschreibung der Erfindung

Die vorstehend genannte Aufgabe wird erfindungs gemäß gelöst durch eine Kapsel, enthaltend Wirkstoffpellets mit mindestens drei verschiedenen Wirkstoffen, die sich hinsichtlich ihres Freisetzungsprofils im Magen-Darm-Trakt unterscheiden, wobei der oder die Wirkstoffe ausgewählt sind aus der Gruppe der Vitamine, Mineralstoffe, Spurenelemente, ungesättigten Fettsäuren, Aminosäuren, pflanzlichen Wirkstoffe und Pflanzenextrakte. Im Gegensatz zur Offenbarung der WO 01/72286, wo in jedem einzelnen Pellet mehrere unterschiedliche Freisetzungsraten verwirklicht sind, verfügt ein bestimmtes Wirkstoffpellet der Erfindung nur über ein Freisetzungsprofil. Das Freisetzungsprofil kann eine schnelle, mittlere und/oder langsame Auflösung des Wirkstoffpellets darstellen. Somit werden Kapseln bereitgestellt, welche mehrere unterschiedliche Pellets enthalten, die jeweils ein unterschiedliches Freisetzungsprofil aufweisen. Damit werden die enthaltenen Wirkstoffe im Magen-Darm-Trakt gezielt dort freigesetzt, wo sie am effektivsten aufgenommen werden.

Gegenstand der Erfindung ist somit eine Kapsel, enthaltend Wirkstoffpellets, die sich hinsichtlich ihres Freisetzungsprofils im Magen-Darm-Trakt unterscheiden, wobei diese Pellets mindestens zwei verschiedene Wirkstoffe enthalten, die ausgewählt sind aus der Gruppe der Vitamine, Mineralstoffe, Spurenelemente, ungesättigten Fettsäuren, Aminosäuren und/oder pflanzlichen Extrakte und Substanzen, wobei mindestens drei Gruppen von Pellets mit jeweils demselben Freisetzungsprofil enthalten sind und die Freisetzung der jeweiligen Wirkstoffe über den gesamten Resorptionsbereich im Magen-Darm-Trakt (Gruppe I), nur im Duodenum oder nur im Duodenum und im Jejunum (Gruppe II),nur im Jejunum, nur im Jejunum und Ileum oder nur im Ileum (Gruppe III) erfolgt.

Die verschiedenen Freisetzungsprofile können durch die Zusammensetzung und/oder den Aufbau der Wirkstoffpellets eingestellt werden. Vorzugsweise werden bestimmte Freisetzungsprofile durch unterschiedliche Beschichtungen der Pellets erreicht, beispielsweise durch Variation der Stärke der Beschichtung und/oder der Auswahl der Zusammensetzung der Beschichtung, um zum Beispiel eine pH-gesteuerte Freisetzung zu nutzen. Typische pharmakologisch und technologisch häufig verwendete Schichten sind Film- und Zuckerbeschichtungen. Beschichtungen mit verzögerter Freisetzung (Retardtabletten) sind zum Beispiel Diffusionsbeschichtungen oder sich auflösende Beschichtungen, wie eine darmlösliche Schicht, d.h. eine Schicht, die gegenüber dem Magensaft im wesentlichen resistent ist, aber während der Passage durch den Darm aufgelöst wird. Geeignete Beschichtungen sind zum Beispiel Schellack, Zein, Gelatine, Celluloseacetatphthalat, Hydroxypropylmethylcellulosephthalat, Ethylcellulose, Stearinsäure, Carnaubawachs, Glycerolbehenat, Polymerisate von Acrylsäureestern und Methacrylsäureestern, wie Eudragit^{®}-Beschichtüngsmaterialien u.a..

In Einzelfällen kann es von Vorteil sein, wenn keine Beschichtung von Zein und Schellack herangezogen wird. Die Beschichtungstechnologie in der Galenik gehört zum Wissen des Fachmanns, so dass weitere Details nicht erläutert werden müssen.

Die vorliegende Erfindung ermöglicht daher, die in den Pellets enthaltenen Wirkstoffe entsprechend der versetzt einsetzenden und ortsgesteuerten Freisetzungen auszuwählen. Beispielsweise können mehrere Pellets dasselbe Freisetzungsprofil aufweisen, so dass eine oder mehrere Gruppen an Pellets vorliegen, die ein schnelles, mittleres und/oder langsames Freisetzungsprofil haben. Auch besteht die Möglichkeit Wirkstoffe über den gesamten Magen-Darm-Trakt oder den gesamten Dünndarm freizusetzen, d.h. eine unmittelbare, schnell ablaufende als auch eine zeitlich verzögerte Freisetzung kann erfolgen. Die obigen Varianten der schnellen, mittleren und langsamen Freisetzung sind daher nicht nur alternativ, sondern auch in Kombination zu verstehen, da bei manchen Wirkstoffen auch mehrere Freisetzungsprofile möglich und erwünscht sind.

"Pellet" soll in der vorliegenden Erfindung jede mögliche Darreichungsform für die orale Verabreichung darstellen, wie Extrudat, Tablette, Teilchen, Perle, Granulat, Dragee und dergleichen, die aufgrund ihrer Größe in Kapseln verabreicht werden kann, ohne dass aufgrund der Größe der umhüllenden Kapsel unerwünschte Probleme beim Schlucken auftreten. Vorzugsweise sind die Pellets Extrudate von Wirk- und Hilfsstoffen.

Die Kapsel der Erfindung dient nur als neutrales Trägermedium, das je nach Bedarfsfall am gewünschten Freisetzungsort im Magen-Darm-Trakt aufgelöst wird, um die enthaltenen Wirkstoffpellets freizusetzen und diesen die entsprechenden Freisetzungsprofile zu ermöglichen. Es kommen sowohl Hart- als auch Weichkapseln in Frage, wobei letztere bevorzugt sind.

Bevorzugt enthaltend die erfindungsgemäßen Kapseln mindestens ein Vitamin, insbesondere mindestens 2 Vitamine, meist bevorzugt 2 bis 10 Vitamine.

Als Beispiel der vorliegenden Erfindung sind die Wirkstoffe aus der Gruppe der Vitamine und Mineralstoffe exemplarisch ausgewählt. Weitere mögliche Inhaltsstoffe stammen aus den Bereichen der Spurenelemente, der ungesättigten Fettsäuren, der Aminosäuren und/oder der pflanzlichen Substanzen und Extrakte. Bei den Vitaminen kommen sowohl wasserlösliche als auch fettlösliche Vitamine in Frage. Geeignete Vitamine sind wasserlösliche Vitamine, wie Vitamin C, z.B. L-(+)-Ascorbinsäure, Calciumascorbat, Kaliumascorbat, 6-Palmitoyl-L-ascorbinsäure; Vitamin B1, z.B. Thiaminhydrochlorid, Thiaminmononitrat; Vitamin B2, z.B. Riboflavin, Riboflavin-5'-phosphatnatrium; Vitamin B6, z.B. Pyridoxinhydrochlorid, Vitamin B 12, z.B. Cyanocobalamin; Vitamin H, z.B. D-Biotin; Folsäure; Vitamin PP (Niacin), z.B. Nicotinamid, Nicotinsäure; Pro-Vitamin B5, z.B. Panthenol (D- und DL-Formen), Ethylpanthenol und Calcium-D-pantothenat. Geeignete fettlösliche Vitamine sind z.B. Vitamin A, wie Vitamin A-palmitat, Vitamin A-acetat, Vitamin A-propinat, trans-Retinol; Vitamin D, z.B. Ergocalciferol, Cholecalciferol, Cholecalciferolcholesterol; Vitamin E, z.B. alpha-Tocopherol, alpha-Tocopherylacetat, alpha-Tocopherylsäuresuccinat (D und DL-Formen); Vitamin K, wie Vitamin K1, z.B. Phytomenadion, und Carotinoide (Provitamin), z.B. Lycopin, Zeaxanthin, Lutein, alpha-Carotin, beta-Carotin, Apocarotinal, gamma-Carotin and beta-Cryptoxanthin. Riboflavin wird bevorzugt in Salzform, wie Riboflavinphosphat verwendet, da die Salzform größere Stabilität zeigt.

Vorzugsweise enthält die erfindungsgemäße Kapsel 20 bis 150, insbesondere 40 bis 120, meist bevorzugt etwa 100 mg Vitamin C, 0,5 bis 2,0, insbesondere 0,8 bis 1,5, meist bevorzugt etwa 1,1 mg Vitamin B1; 0,8 bis 2,5, insbesondere 1,0 bis 2,0, meist bevorzugt etwa 1,2 mg Vitamin B2, 0,8 bis 2,5, insbesondere 1,0 bis 2,0, meist bevorzugt etwa 1,5 mg Vitamin B6, 0,8 bis 5,0, insbesondere 1,2 bis 3,5, meist bevorzugt etwa 3,0 µg Vitamin B 12, 5 bis 200 insbesondere 10 bis 100, meist bevorzugt etwa 50 µg D-Biotin; 50 bis 600 insbesondere 100 bis 500, meist bevorzugt etwa 400 µg Folsäure; 5,0 bis 50,0, insbesondere 10 bis 30, meist bevorzugt etwa 16 mg Vitamin PP (Niacin); 1,0 bis 50,0, insbesondere 2 bis 20, meist bevorzugt etwa 6 mg Panthenol, 300 bis 1300 insbesondere 500 bis 1000, meist bevorzugt etwa 800 µg Vitamin A, 0 bis 20 insbesondere 2,5 bis 15,0, meist bevorzugt etwa 5 µg, Vitamin D, 0,1 bis 300,0, insbesondere 5,0 bis 15,0, meist bevorzugt etwa 12 mg Vitamin E, und 10 bis 200 insbesondere 25 bis 150, meist bevorzugt etwa 75 µg Vitamin K.

Nach einer besonders bevorzugten Ausführungsform der Erfindung unterscheidet man bei den Wirkstoffen bzw. Nährstoffen drei Gruppen, d.h. den Wirkstoffen, die über den gesamten Resorptionsbereich des Magen-Darm-Trakts (Gruppe I) freigesetzt werden, den Wirkstoffen, die im Duodenum oder im Duodenum und im Jejunum freigesetzt werden (Gruppe II) und denjenigen, deren Freisetzung im Jejunum, im Jejunum und Ileum oder im Ileum erfolgt (Gruppe III). Entsprechend dieser Einteilung, die den optimalen Resorptionsort für einen ausgewählten Wirkstoff repräsentiert, erfolgt die Zuordnung des jeweiligen Wirkstoffes vorteilhafterweise zu den jeweiligen Pellets, die über ein entsprechendes Freisetzungsprofil verfügen, um die Wirkstoffe an den gewünschten Resorptionsort zu bringen.

Dieses Prinzip soll am Beispiel der Vitamine verdeutlicht werden. Beispielweise sind Vitamine der Gruppe I ausgewählt aus Niacin, Pantothensäure, Vitamin D und Biotin, Vitamine der Gruppe II ausgewählt aus Vitamin A, Riboflavin, Thiamin und Folsäure und Vitamine der Gruppe III ausgewählt aus Vitamin B6, Vitamin C und Vitamin K. Dies stellt selbstverständlich keine abschließende Auflistung dar, sondern dient lediglich der Veranschaulichung. Weitere Beispiele sind dem Fachmann ohne weiteres geläufig.

Nach dieser Variante der Erfindung enthält jedes Wirkstoffpellet entweder ein oder mehrere Vitamine der Gruppe I, II oder III. Es können aber auch Kombinationen bereitgestellt werden. Da die Vitamine der Gruppe I über den gesamten Bereich freigesetzt und dort resorbiert werden sollen, besteht auch die Möglichkeit, dass zwei Typen von Wirkstoffpellets vorhanden sind, wobei der eine Typ Vitamine der Gruppe I und II und der andere Typ Vitamine der Gruppe I und III enthält. Damit liegen die im gesamten Bereich freizusetzenden Vitamine sowohl in den Pellets mit dem eher unmittelbaren, d.h. schnellen Freisetzungsprofil vor als auch in denjenigen mit einem eher mittleren und/oder langsamen Freisetzungsprofil.

Eine "schnelle" Freisetzung bedeutet, dass die gewünschte Plasmakonzentration des Vitamins innerhalb relativ kurzer Zeit erreicht wird, beispielsweise etwa 0,5 bis 1 Stunde nach der Einnahme bzw. bei im Dünndarm resorbierten Wirkstoffen nach der Einnahme und der Magenpassage, wobei im wesentlichen der Gesamtgehalt des Vitamins eines Pellets auf einmal freigesetzt wird. Die Freisetzung und Resorption findet dabei hauptsächlich im Magen bzw. im oberen Teil des Dünndarms statt, wobei hierfür auch die Menge und Art der zuvor aufgenommenen Nahrung und der zeitliche Rahmen, wann das letzte Mal gegessen wurde, von Bedeutung sind. Die Begriffe "schnelle", "mittlere" und "langsame" Freisetzung sind demnach relative Begriffe und dienen als Parameter, aber stellen keine absoluten Werte dar.

Die Zuordnung der Wirkstoffe, wie zum Beispiel der Vitamine zu den gewünschten Freisetzungsprofilen ist ausschlaggebend für den Resorptionsort, an dem die Freisetzung dann erfolgt, so dass hierbei sehr sorgfältig vorgegangen werden sollte. Ein falscher Freisetzungsort setzt selbstverständlich das Wirkungspotential des Wirkstoffs um ein erhebliches Maß herab.

Gegenstand der Erfindung ist auch ein Verfahren zur Herstellung der erfindungsgemäßen Kapseln, umfassend
- Auswählen der Wirkstoffe in Form von z.B. Vitaminen, Mineralstoffen, Spurenelementen, ungesättigten Fettsäuren, Aminosäuren oder pflanzlicher Substanzen bzw. Extrakte anhand ihres jeweiligen Resorptionsorts;
- Herstellen von mehreren Gruppen von Wirkstoffpellets mit unterschiedlichen Freisetzungsprofilen für die jeweiligen Resorptionsorte unter Einbringen der Vitamine und optional weiterer Wirkstoffe, die entsprechend den Freisetzungsprofilen und Resorptionsorten auf die Gruppen der Wirkstoffpellets verteilt werden und
- Einbringen der Wirkstoffpellets in eine Kapsel.

Der oder die Wirkstoffe in Form von einem oder mehreren Wirkstoffen, liegen in den Wirkstoffpellets der Erfindung vorzugsweise in einer Menge von etwa 0,001 bis etwa 99 Gew.-%.

Die in den erfindungsgemäßen Kapseln enthaltenen Wirkstoffpellets können neben den oben geschilderten Vitaminen weitere Wirkstoffe enthalten, wie Mineralstoffe, Spurenelemente, Pflanzenextrakte, Aminosäuren und ungesättigte Fettsäuren.

Beispiele für Mineralstoffe umfassen Calcium, wie Calciumhydrogenphosphat, Calciumcitrat; Magnesium, wie Magnesiumcarbonat, Magnesiumlactat; Kalium, wie Kaliumchlorid, Kaliumsulfat; Phosphor, wie Calciumphosphat; und Chlorid, wie Kaliumchlorid, Magnesiumchlorid. Geeignete Spurenelemente können sowohl anorganische als auch organische Salze sein. Beispielhaft seien genannt: Eisen, wie Eisenfumarat, Eisencitrat, Eisenlactat; Zink, wie Zinklactat, Zinkcitrat, Zinkoxid; Jod, wie Natriumjodat, Kaliumjodid; Kupfer, wie Kupfergluconat, Kupfersulphat; Mangan, wie Mangancitrat, Mangansulfat; Molybdän, wie Natriummolybdat, Ammoniummolybdat; Selenium, wie Natriumselenat, Natriumselenit; Chrom, wie Chromchlorid, Chromcitrat; Silicate, wie Siliciumdioxid, Natriumsilicat; und Fluorid, wie Natriumfluorid. Als Mineralien und Spurenelemente sind erfindungsgemäß Calcium, Magnesium, Eisen, Zink, Kupfer, Mangan, Selen, Phosphor und Jod, deren Salze und Mischungen hiervon besonders bevorzugt.

Vorzugsweise enthält die erfindungsgemäße Kapsel ein oder mehrere Mineralstoffpellets wobei eines oder mehrere der folgenden Mineralien in den jeweils angegebenen Mengen enthalten ist:

100 bis 2500, insbesondere 500 bis 1200, meist bevorzugt etwa 1000 mg Calcium, 50 bis 1000, insbesondere 100 bis 600, meist bevorzugt etwa 375 mg Magnesium, 500 bis 5000, insbesondere 1500 bis 3000, meist bevorzugt etwa 2000 mg Kalium, 100 bis 2000, insbesondere 300 bis 1500, meist bevorzugt etwa 700 mg Phosphor, 500 bis 7500, insbesondere 750 bis 5000, meist bevorzugt etwa 800 mg Chlorid, 0,5 bis 50, insbesondere 1,0 bis 20, meist bevorzugt etwa 2,0 mg Mangan, 5,0 bis 50, insbesondere 7,5 bis 25, meist bevorzugt etwa 5,0 mg Eisen; 1,0 bis 50, insbesondere 2,5 bis 20, meist bevorzugt etwa 5,0 mg Zink; 10 bis 500, insbesondere 100 bis 200, meist bevorzugt etwa 150 µg Jod, 0,1 bis 5,0, insbesondere 0,5 bis 5,0, meist bevorzugt etwa 1,0 mg Kupfer, 0,5 bis 15,0, insbesondere 1,0 bis 12,5, meist bevorzugt etwa 2,0 mg Mangan, 10 bis 500, insbesondere 20 bis 450, meist bevorzugt etwa 50 µg Molybdän, 5 bis 250, insbesondere 10 bis 200, meist bevorzugt etwa 55 µg Selen, 5 bis 200, insbesondere 10 bis 150, meist bevorzugt etwa 40 µg Chrom, und 0,5 bis 15,0, insbesondere 1,0 bis 10,0, meist bevorzugt etwa 3,5 mg Fluorid.

Einsetzbare Pflanzenextrakte sind vorzugsweise ausgewählt aus *Ginkgo biloba*, *Panax Ginseng, Vitis vinifera*, d.h. Weinlaubblätter als auch Traubenkerne, *Guarana, Cimicifuga racemosa und Turnera aphrodisiaca*, d.h. Damiana Blätter oder pflanzlichen Spezialextrakten, die reich an Kaempferol bzw. Kaempferolglukosiden und/ oder reich an Lutein bzw. anderen Flavonoiden sind. Je nach der gewünschten Verwendung können ebenfalls Aminosäuren zugesetzt werden, wobei Lysin, Arginin und Taurin insbesondere bevorzugt sind. Auch ungesättigte Fettsäuren können enthalten sein, wie ω-Fettsäuren, insbesondere DHA.

Die in den Kapseln der Erfindung enthaltenen Wirkstoffpellets sind derart zusammengestellt, dass die übliche Einzeldosis eines Nährstoffes, beispielsweise verteilt auf mehrere Wirkstoffpellets oder in einem Wirkstoffpellet enthalten, im Bereich von etwa 10 bis 300 %, bevorzugt etwa 100 % der empfohlenen Tagesdosis liegt. Die zusätzliche Menge an vorhandenen Mineralien und Spurenelementen liegt vorzugsweise im Bereich von etwa 1 bis 100 % der empfohlenen Tagesdosis.

Die Wirkstoffpellets der vorliegenden Erfindung können weitere Hilfsstoffe aufweisen, wie Weichmacher, z.B. Glycerol, Polyethylenglycol, Castoröl und acetylierte Monoglyceride; pH-Modifizierungsmittel, wie Kaliumhydrogenphosphat; Füllstoffe, wie Calciumcarbonat; Bindemittel, wie mikrokristalline Cellulose, Stabilisatoren, Schmiermittel, Sprengmittel, Zerfallsmittel, Trennmittel, Farbstoffe und dergleichen.

Die mit der vorliegenden Erfindung verbundenen Vorteile sind vielschichtig:
Die Verabreichung zahlreicher Tabletten mit unterschiedlichen Freisetzungscharakteristika wird vermieden. Dies kommt um so mehr bei Multikomponent-Formulierungen zum Tragen, bei denen eine Vielzahl an Wirkstoffen zum Einsatz kommt. Zum Beispiel kann ein als Nahrungsergänzungsmittel verabreichtes Multivitaminpräparat 10 Vitamine, 5 Mineralstoffe und 12 Spurenelemente enthalten, die schnell freigesetzt werden sollen, und eine gleiche Anzahl Wirkstoffe, die langsamer freigesetzt werden sollen. Dies umfasst 54 verschiedene Wirkstoffe mit unterschiedlichen Freisetzungscharakteristika, was bei einer jeweiligen Einzeldosierung den Verbraucher völlig überfordern würde.

Die Einnahme der erfindungsgemäßen Multikomponenten-Kapsel, enthaltend verschiedene Vitamine und andere Wirkstoffe bzw. Nährstoffe, ist für den Verbraucher oder Patienten daher sehr bequem und komfortabel, die tägliche Einnahme wird auf eine oder wenige Kapseln reduziert. Ferner erfolgt die tatsächliche Aufnahme der Wirkstoffe nahezu unbeeinflusst voneinander. Die enthaltenen Wirkstoffe, insbesondere die Vitamine und andere Nährstoffe sowie Pflanzenextrakte und -substanzen, werden im Magen-Darm-Trakt dort freigesetzt, wo sie am effektivsten aufgenommen werden, d.h. deren Bioverfügbarkeit wird deutlich verbessert. Durch die gezielte Freisetzung der unterschiedlichen Wirkstoffe kann deren Dosierung sehr viel genauer eingestellt werden, wodurch die Wirkstoffmenge reduziert, d.h. optimiert werden kann, und Überdosierungen vermieden werden. Durch die erfindungsgemäße gezielte Freisetzung kann jeder Wirkstoff an den gewünschten Resorptionsort befördert werden, wo dieser sein optimales Wirkstoffspektrum entfalten kann.

Ferner können auch Wirkstoffe zusammen verabreicht werden, die üblicherweise miteinander nicht kompatibel oder verträglich sind, da diese getrennt voneinander vorliegen. So sind einige Spurenelemente und Mineralien zusammen mit einigen fettlöslichen Vitaminen unverträglich. Diese können erfindungsgemäß zusammen verabreicht werden.

Die vorliegende Erfindung stellt daher eine neuartige Darreichungsform zur Verfügung, die auch bei einer größeren Anzahl an Wirkstoffen eine optimale Bioverfügbarkeit für jeden einzelnen Wirkstoff liefert.

### Beschreibung der Figuren

Die beigefügten Figuren veranschaulichen die vorliegende erfindungsgemäße Lehre ohne diese darauf zu beschränken. Im Einzelnen zeigen:
Figur 1 eine schematische Darstellung einer Darreichungsform aus dem Stand der Technik nach der WO 01/72286;
Figur 2 eine schematische Darstellung einer erfindungsgemäßen Ausführungsform einer Kapsel der Erfindung,
Figur 3 die Resorption von ausgewählten Vitaminen an verschiedenen Bereichen im Dünndarm
Figur 4 vier Ausführungsformen von Kapseln aus dem Stand der Technik sowie zwei erfindungsgemäße Ausführungsformen einer Kapsel, und
Figur 5 drei verschiedene Ausführungsformen von Pellets für eine erfindungsgemäße Kapsel.

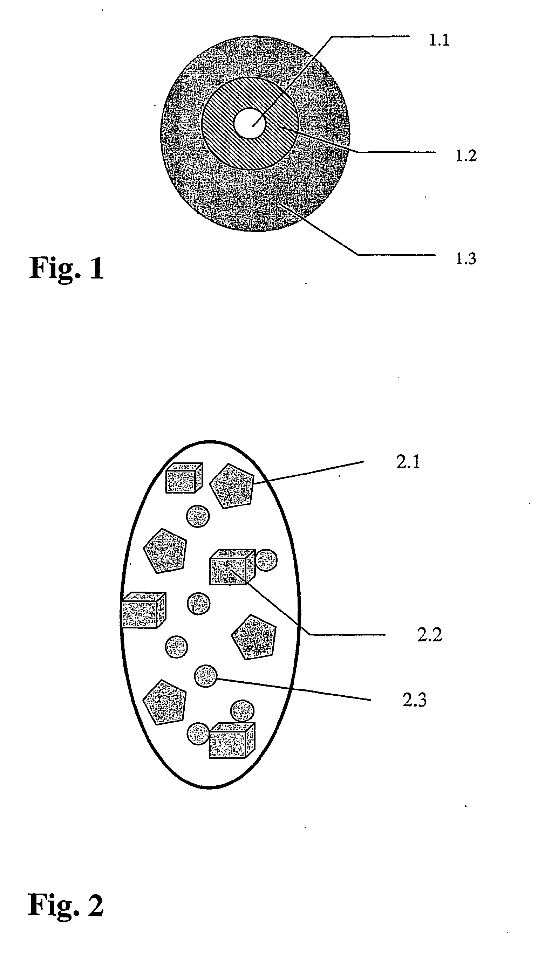

Figur 1 zeigt schematisch eine Darreichungsform gemäß der WO 01/72286 im Schnitt; in der jeder einzelne Pellet Schichten mit unterschiedlichen Freisetzungsraten aufweist. Dargestellt ist ein Kern 1.1 mit einer langsamen Freisetzungsrate, eine äußere Schicht 1.3 mit einer schnellen Freisetzungsrate und eine Zwischenschicht 1.2. Es handelt sich demnach um eine Darreichungsform, bei der in jedem einzelnen Pellet mehrere unterschiedliche Freisetzungsraten verwirklicht sind.

Figur 2 veranschaulicht eine Ausführungsform einer Kapsel der Erfindung. Schematisch sind anhand verschiedener Symbole drei verschiedene Gruppen von Wirkstoffpellets dargestellt, die jeweils andere Freisetzungsprofile aufweisen. So repräsentieren die Fünfringe mit der Bezugsziffer 2.1 Pellets mit einer langsamen Freisetzungsrate, die mit 2.2 bezeichneten Quader sind Pellets mit einer mittleren Freisetzungsrate und die mit 2.3 gekennzeichneten Kreise stellen Pellets mit einer schnellen Freisetzungsrate dar. Die verschiedenen Wirkstoffpellets der drei Gruppen werden zu drei verschiedenen Resorptionsorten befördert, wo der oder die enthaltenen Wirkstoffe freigesetzt und resorbiert werden und ihr optimales Wirkungsspektrum entfalten können.

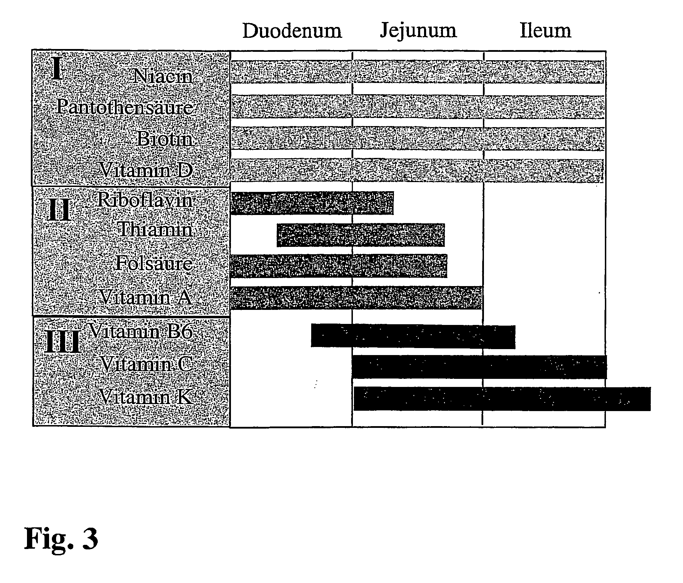

In Figur 3 ist der optimale Resorptionsort oder -bereich für ausgewählte Vitamine im jeweilgen Dünndarmbereich dargestellt. Beispielsweise zeigt sich, dass Niacin, Pantothensäure und Biotin über den gesamten Bereich verfügbar sein sollen, wodurch sich eine erfindungsgemäße Zuordnung in die Gruppe I, wie bereits erläutert, ergibt. Entsprechend können Vitamin C, Thiamin, Folsäure und Vitamin A, die im Duodenum und Jejenum resorbiert werden der Gruppe II zugeordnet werden. Die Vitamine der Gruppe III, die hauptsächlich im Jejenum und Ileum resorbiert werden sind die hier dargestellten Vitamin B6, Riboflavin, Vitamin D, Vitamine B 12 und Vitamin K. Bei Vitamin B6 zeigt sich, dass der optimale Resorptionsbereich eigentlich im Jejenum und Ileum liegt, sich aber auch bis zum Duodenum reichen kann, sich also auch auf Zwischenbereiche erstreckt. Dies kann bei den Freisetzungsprofilen Berücksichtigung finden.

Die in Figur 2 dargestellten Wirkstoffpellets 1 können daher ein oder mehrere Vitamine aus der Gruppe I, die Wirkstoffpellets 2 ein oder mehrere Vitamine der Gruppe II und die Wirkstoffpellets 3 ein oder mehrere Vitamine aus der Gruppe III, gegebenenfalls zusammen mit Mineralien und Spurenelementen sowie weiteren Hilfsstoffen enthalten. Auch können die in Figur 2 dargestellten Wirkstoffpellets 1 ein oder mehrere Vitamine aus der Gruppe I und II und die Wirkstoffpellets 2 ein oder mehrere Vitamine der Gruppe I und III enthalten. Eine Vielzahl von Varianten und Kombinationen ist möglich insbesondere, wenn weitere Wirk- und Nährstoffe (Mineralstoffe, Aminosäuren, ungesättigte Fettsäuren) sowie pflanzliche Extrakte und Substanzen enthalten sind.

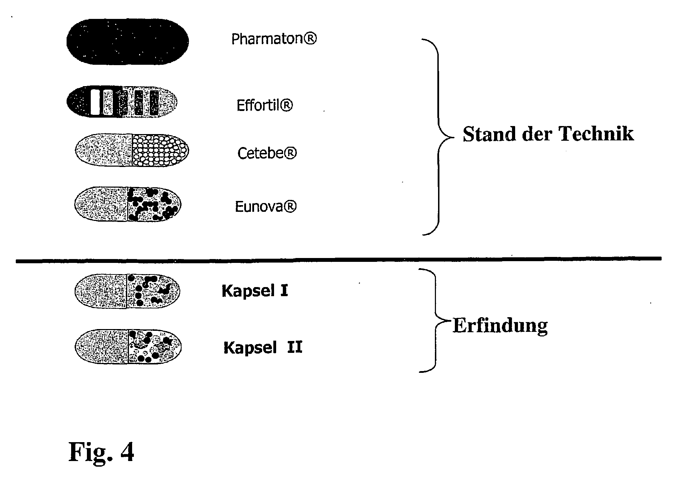

Figur 4 zeigt vier aus dem Stand der Technik bekannte Ausführungsformen, die aufgrund der gewählten Darreichungsform eine entsprechende Langzeitwirkung zeigen. Bei der obersten abgebildeten Kapsel, den Pharmaton^{®}Vital-Kapseln, ein Multivitamin-Mineralstoff-Präparat mit *Ginseng*, und der zweiten abgebildeten Kapsel, dem Kreislaufmittel Effortil^{®} in Form von Depot Perlongetten, beides erhältlich von der Fa. Boehringer Ingelheim GmbH, wird die Langzeitwirkung durch entsprechende äußere Beschichtung der Kapsel erreicht. In der zweiten und dritten dargestellten Kapsel, dem Vitamin-C-Präparat Cetebe^{®} und der Eunova^{®} Multivitamin-Kapsel, beides erhältlich von GlaxoSmithHline Consumer Healthcare GmbH & Co. KG, werden dagegen mehrschichtige Pellets, wie dies beispielsweise in der WO 01/72286 beschrieben ist (siehe auch Figur 1), eingesetzt, um die Depot- oder Langzeitwirkung zu erhalten.

Weiterhin sind in Figur 4 zwei erfindungsgemäße Ausführungsformen von Kapseln dargestellt, welche die verschiedenen Wirkstoffpellets mit unterschiedlichen Freisetzungsprofilen in einer Kapsel zeigen. Kapsel I zeigt zwei Gruppen von Wirkstoffpellets und Kapsel II zeigt drei Gruppen von Wirkstoffpellets mit verschiedenen Freisetzungsprofilen. Die Freisetzungsprofile sind daher bei den unterschiedlichen Gruppen zeitversetzt. Durch die kontrollierte Freisetzung gezielt am gewünschten Resorptionsort wird eine optimale Dosierung möglich, die zu einer optimalen Resorption jedes einzelnen Wirkstoffs führt.

Die nachfolgenden Beispiele dienen der Illustration erfindungsgemäßer Formulierungen. Sie sind lediglich als mögliche, exemplarisch dargestellte Vorgehensweisen zu verstehen, ohne die Erfindung auf deren Inhalt zu beschränken.

### Beispiele

Für die Passage des GIT werden in der neueren Literatur folgende Transitzeiten und pH-Werte angenommen (Dressman, 2003):

| | **pH-Wert (nüchtern)** | **Transitzeit** |
|---|---|---|
| Magen | 1-3 | 1-2 Stunden (Tabletten) |
| | | 30 Minuten (Pellets) |
| Duodenum | 6,0 | 3-4 Stunden |
| Jejunum | 6,5-6,8 | |
| Ileum | 7,2-7,5 | |

Aufgrund dieser physiologischen Gegebenheiten und Ihren Vorgaben ("Absorption of vitamins in the small intestine") schlagen wir vor, die Inhaltsstoffe auf drei Pelletsorten aufzuteilen:

### Pelletsorte 1: Mineralstoffpellets

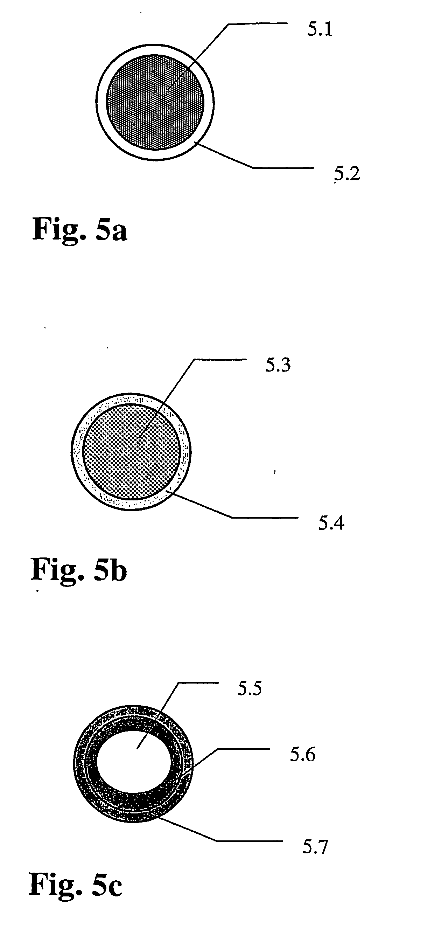

Aus Stabilitätsgründen ist eine Abtrennung der Mineralien von den Vitaminen vorteilhaft. Der schematische Aufbau eines solchen Pellets ist in Figur 5a dargestellt.
Der Mineralstoff-haltige Pelletkern 5.1 wird im Extrusionsverfahren hergestellt und kann optional mit einem farbigen Schutzfilm 5.2 aus Schellack versehen werden. Das Coating ist nicht funktional. Wahlweise kann auch durch eine Erhöhung der Schichtdicke des Schellack-Coatings eine Magensaftresistenz der Pellets erzielt werden.

### Pelletsorte 2: Vitaminpellets Gruppe I+II

Der schematische Aufbau dieser Pelletgruppe ist in Figur 5b dargestellt.
Die Wirkstoffgruppen (hier beispielhaft: Vitamingruppen) I und II (z.B. Niacin, Vit. A, Thiamin, Folsäure etc.) sollen über den gesamten Bereich des Dünndarms bzw. bevorzugt bis zum Jejunum freisetzen. Hierfür ist ein magensaftresistenter (MSR) Filmüberzug 5.4 erforderlich, der über 1-2h ein Eindringen von Säure in den Pelletkern 5.3 verhindert und nach Erreichen des Duodenums beginnt sich aufzulösen und die Vitamine zu liberieren. Beispielhaft weisen die Vitamingruppen I und II folgende Zusammensetzungen auf:

| | | |
|---|---|---|
| Gruppe I: | Niacin | 16 mg |
| | Pantothensäure | 6 mg |
| | Biotin | 50 µg |
| | Vitamin D | 5 µg |
| | | |
| Gruppe II: | Riboflavin (Vit. B2) | 1,2 mg |
| | Vitamin A | 800 (µg Retinol equivalents) |
| | Thiamin | 1,1 mg |
| | Folsäure | 400 µg |

Der Pelletkern wird ebenfalls im Extrusionsverfahren hergestellt und anschließend mit Schellack magensaftresistent überzogen. Die Überprüfung des Freisetzungsverhaltens wird in Anlehnung an die entsprechenden Arzneibuchmonographien durchgeführt.

### Pelletsorte 3: Vitaminpellets Gruppe III

Der schematische Aufbau dieser Pelletgruppe ist in Figur 5b dargestellt.
Pelletsorte 3 sollte ihre Inhaltsstoffe erst ab dem Jejunum oder später freigeben.
Dies macht eine Retardmembran 5.6 unterhalb des MSR-Überzugs 5.7 erforderlich, die den Eintritt der Wirkstofffreigabe und auch die Wirkstofffreigabe selbst verzögert. Diese kann aus Stoffen wie Stearinsäure, Carnaubawachs, Glycerolbehenat o.ä. aufgebaut sein. Der Pelletkern 5.5 wird ebenfalls im Extrusionsverfahren hergestellt und weist beispielhaft folgende Zusammensetzung auf:

| | | |
|---|---|---|
| Gruppe III: | Vitamin B6 | 1,5 mg |
| | Vitamin C | 100 mg |
| | Vitamin K | 75 µg |

Alternativ ist auch eine Matrix mit diesen lipophilen Zuschlagsstoffen bzw. eine einfach Erhöhung der Filmdicke des MSR-Überzugs denkbar.
Die Einstellung und Überprüfung des Freisetzungsverhaltens wird ebenfalls gemäß EuAB oder USP durchgeführt.

### Pelletmischung und Kapselabfüllung

Die drei resultierenden, unterschiedlich gefärbten, Pelletsorten werden miteinander gemischt und in eine Hartgelatinekapsel der Größe 0 oder 0el (alternativ 2x Größe 1) abgefüllt.
Die genaue qualitative und quantitative Zusammensetzung der Vitamine und Mineralstoffe kann den jeweiligen Bedürfnissen bzw. Erfordernissen flexibel angepasst werden.

## Patentansprüche

1. Kapsel, enthaltend Wirkstoffpellets, die sich hinsichtlich ihres Freisetzungsprofils im Magen-Darm-Trakt unterscheiden, wobei diese Pellets mindestens zwei verschiedene Wirkstoffe enthalten, die ausgewählt sind aus der Gruppe der Vitamine, Mineralstoffe, Spurenelemente, ungesättigten Fettsäuren, Aminosäuren und/oder pflanzlichen Extrakte und Substanzen, **dadurch gekennzeichnet, dass** mindestens drei Gruppen von Pellets mit jeweils demselben Freisetzungsprofil enthalten sind und die Freisetzung der jeweiligen Wirkstoffe über den gesamten Resorptionsbereich im Magen-Darm-Trakt: (Gruppe I), im Duodenum oder im Duodenum und im Jejunum (Gruppe II), im Jejunum, im Jejunum und Ileum oder im Ileum (Gruppe III) erfolgt.

2. Kapsel nach Anspruch 1, **dadurch gekennzeichnet, dass** die unterschiedlichen Freisetzungsprofile eine schnelle, mittlere und/oder langsame Auflösung der Wirkstoffpellets darstellen.

3. Kapsel nach Anspruch 1 oder 2, **dadurch gekennzeichnet, dass** die Wirkstoffpellets beschichtet sind und die Stärke und/oder Zusammensetzung der Beschichtung die verschiedenen Freisetzungsprofile bestimmen.

4. Kapsel nach Anspruch 1, 2 oder 3, **dadurch gekennzeichnet, dass** jedes Pellet ein ausgewähltes Freisetzungsprofil aufweist und die enthaltenen Vitamine, Mineralstoffe, Spurenelemente, ungesättigten Fettsäuren, Aminosäuren und/oder pflanzlicher Extrakte und Substanzen entsprechend des Freisetzungsprofils ausgewählt sind.

5. Kapsel nach einem der vorangehenden Ansprüche, **dadurch gekennzeichnet, dass** die Vitamine der Gruppe I ausgewählt sind aus Niacin, Pantothensäure, Biotin und Vitamin D.

6. Kapsel nach einem der vorangehenden Ansprüche, **dadurch gekennzeichnet, dass** die Vitamine der Gruppe II ausgewählt sind aus Vitamin A, Thiamin, Vitamin B2 und Folsäure.

7. Kapsel nach einem der vorangehenden Ansprüche, **dadurch gekennzeichnet, dass** die Vitamine der Gruppe III ausgewählt sind aus Vitamin B6, Vitamin C und Vitamin K.

8. Kapsel nach einem der vorangehenden Ansprüche, **dadurch gekennzeichnet, dass** jedes Wirkstoffpellet entweder Vitamine und/oder Mineralstoffe und/oder Spurenelemente und/oder Aminosäuren und/oder ungesättigte Fettsäuren und/oder pflanzliche Extrakte / Substanzen der Gruppe I, II oder III enthält.

9. Kapsel nach einem der vorangehenden Ansprüche, **dadurch gekennzeichnet, dass** zwei Typen von Wirkstoffpellets vorhanden sind, wobei ein Typ Vitamine, Mineralstoffe, Spurenelemente, ungesättigte Fettsäuren, Aminosäuren und/oder pflanzlicher Extrakte und Substanzen der Gruppe I und II und der andere Typ aus der Gruppe I und III enthält.

10. Kapsel nach einem der vorangehenden Ansprüche, **dadurch gekennzeichnet, dass** die Mineralstoffe und Spurenelemente ausgewählt sind aus Calcium, Magnesium, Eisen, Zink, Kupfer, Kalium, Mangan, Selen, Chrom, Fluorid, Phosphor und Jod, deren Salzen und Mischungen hiervon.

11. Kapsel nach einem der vorangehenden Ansprüche, **dadurch gekennzeichnet, dass** die Pflanzenextrakte ausgewählt sind aus *Ginkgo biloba, Panax Ginseng, Vitis vinifera, Guarana, Cimicifuga racemosa, Turnera aphrodisiaca*, pflanzliche Spezialextrakte reich an Kaempferol bzw. Kaempferolglukosiden und/ oder reich an Lutein bzw. anderen Flavonoiden.

12. Kapsel nach einem der vorangehenden Ansprüche, **dadurch gekennzeichnet, dass** die Aminosäuren ausgewählt sind aus Lysin, Arginin und Taurin.

13. Kapsel nach einem der vorangehenden Ansprüche, **dadurch gekennzeichnet, dass** die ungesättigten Fettsäuren ω-Fettsäuren darstellen.

14. Verfahren zur Herstellung der Kapseln nach einem der Ansprüche 1 bis 13, umfassend
- Auswählen der Wirkstoffe anhand ihres jeweiligen Resorptionsorts;
- Herstellen von mindestens drei Gruppen von Wirkstoffpellets mit unterschiedlichen Freisetzungsprofilen für die jeweiligen Resorptionsorte unter Einbringen der Vitamine und optional weiterer Wirkstoffe; die entsprechend den Freisetzungsprofilen und Resorptionsorten auf die Gruppen der Wirkstoffpellets verteilt werden und
- Einbringen der Wirkstoffpellets in eine Kapsel.

## Claims

1. Capsule containing active substance pellets which differ in their release profile in the gastro-intestinal tract, these pellets containing at least two different active substances which are selected from among the vitamins, minerals, trace elements, unsaturated fatty acids, amino acids and/or plant extracts and substances, **characterised in that** at least three groups of pellets each having the same release profile are contained and the release of the particular active substance takes place over the entire absorption range in the gastro-intestinal tract (group I), in the duodenum or in the duodenum and in the jejunum (group II), or in the jejunum, in the jejunum and ileum or in the ileum (group III).

2. Capsule according to claim 1, **characterised in that** the different release profiles represent rapid, moderate and/or slow dissolving of the active substance pellets.

3. Capsule according to claim 1 or 2, **characterised in that** the active substance pellets are coated and the thickness and/or composition of the coating determine the different release profiles.

4. Capsule according to claim 1, 2 or 3, **characterised in that** each pellet has a selected release profile and the vitamins, minerals, trace elements, unsaturated fatty acids, amino acids and/or plant extracts and substances contained therein are selected in accordance with the release profile.

5. Capsule according to one of the preceding claims, **characterised in that** the vitamins of group I are selected from niacin, pantothenic acid, biotin and vitamin D.

6. Capsule according to one of the preceding claims, **characterised in that** the vitamins of group II are selected from vitamin A, thiamine, vitamin B2 and folic acid.

7. Capsule according to one of the preceding claims, **characterised in that** the vitamins of group III are selected from vitamin B6, vitamin C and vitamin K.

8. Capsule according to one of the preceding claims, **characterised in that** each active substance pellet contains either vitamins and/or minerals and/or trace elements and/or amino acids and/or unsaturated fatty acids and/or plant extracts / substances of group I, II or III.

9. Capsule according to one of the preceding claims, **characterised in that** two types of active substance pellets are present, one type comprising vitamins, minerals, trace elements, unsaturated fatty acids, amino acids and/or plant extracts and substances of groups I and II and the other type comprising substances of groups I and III.

10. Capsule according to one of the preceding claims, **characterised in that** the minerals and trace elements are selected from calcium, magnesium, iron, zinc, copper, potassium, manganese, selenium, chromium, fluoride, phosphorus and iodine, the salts thereof and mixtures thereof.

11. Capsule according to one of the preceding claims, **characterised in that** the plant extracts are selected from *Ginkgo biloba, Panax Ginseng, Vitis vinifera, Guarana, Cimicifuga racemosa, Turnera aphrodisiaca*, special plant extracts rich in kaempferol or kaempferol glucosides and/ or rich in lutein or other flavonoids.

12. Capsule according to one of the preceding claims, **characterised in that** the amino acids are selected from lysine, arginine and taurine.

13. Capsule according to one of the preceding claims, **characterised in that** the unsaturated fatty acids are ω-fatty acids.

14. Process for preparing the capsules according to one of claims 1 to 13, comprising
- selecting the active substances on the basis of their respective absorption sites;
- producing at least three groups of active substance pellets with different release profiles for the respective absorption sites, incorporating the vitamins and optionally other active substances which are divided between the groups of active substance pellets according to their release profiles and absorption sites, and
- introducing the active substance pellets into a capsule.

## Revendications

1. Capsule comprenant des pellets de principe actif, lesquels se distinguent au niveau de leur profil de libération dans le tractus gastro-intestinal, ces pellets contenant au moins deux principes actifs différents qui sont choisis dans le groupe des vitamines, des minéraux, des oligo-éléments, des acides gras insaturés, des acides aminés et/ou des substances et extraits végétaux, **caractérisée en ce qu'a**u moins trois groupes de pellets ayant chacun le même profil de libération sont contenus et **en ce que** les principes actifs respectifs sont libérés sur toute la zone de résorption dans le tractus gastro-intestinal (groupe I), dans le duodénum ou dans le duodénum et le jéjunum (groupe II), dans le jéjunum, dans le jéjunum et l'iléon ou dans l'iléon (groupe III).

2. Capsule selon la revendication 1, **caractérisée en ce que** les différents profils de libération représentent une dissolution rapide, moyenne et/ou lente des pellets de principes actifs.

3. Capsule selon la revendication 1 ou 2, **caractérisée en ce que** les pellets de principes actifs sont enrobés et **en ce que** l'épaisseur et/ou la composition de l'enrobage détermine(nt) les différents profils de libération.

4. Capsule selon la revendication 1, 2 ou 3, **caractérisée en ce que** chaque pellet présente un profil de libération choisi et **en ce que** les vitamines, minéraux, oligo-éléments, acides gras insaturés, acides aminés et/ou substances et extraits végétaux contenus sont choisis en fonction du profil de libération.

5. Capsule selon l'une quelconque des revendications précédentes, **caractérisée en ce que** les vitamines du groupe 1 sont choisies parmi la niacine, l'acide pantothénique, la biotine et la vitamine D.

6. Capsule selon l'une quelconque des revendications précédentes, **caractérisée en ce que** les vitamines du groupe II sont choisies parmi la vitamine A, la thiamine, la vitamine B2 et l'acide folique.

7. Capsule selon l'une quelconque des revendications précédentes, **caractérisée en ce que** les vitamines du groupe III sont choisies parmi la vitamine B6, la vitamine C et la vitamine K.

8. Capsule selon l'une quelconque des revendications précédentes, **caractérisée en ce que** chaque pellet de principes actifs contient soit des vitamines et/ou des minéraux et/ou des oligo-éléments et/ou des acides aminés et/ou des acides gras insaturés et/ou des substances/extraits végétaux du groupe I, II ou III.

9. Capsule selon l'une quelconque des revendications précédentes, **caractérisée en ce que** deux types de pellets de principes actifs sont présents, un type contenant des vitamines, des minéraux, des oligo-éléments, des acides gras insaturés, des acides aminés et/ou des substances et extraits végétaux du groupe I et II, et l'autre type contenant des vitamines, des minéraux, des oligo-éléments, des acides gras insaturés, des acides aminés et/ou des substances et extraits végétaux du groupe I et III.

10. Capsule selon l'une quelconque des revendications précédentes, **caractérisée en ce que** les minéraux et les oligo-éléments sont choisis parmi le calcium, le magnésium, le fer, le zinc, le cuivre, le potassium, le manganèse, le sélénium, le chrome, le fluorure, le phosphore et l'iode, leurs sels et leurs mélanges.

11. Capsule selon l'une quelconque des revendications précédentes, **caractérisée en ce que** les extraits végétaux sont choisis parmi le *Ginkgo biloba*, le *Panax ginseng*, la *Vitis vinifera*, le Guarana, le *Cimicifuga racemosa*, la *Turnera aphrodisiaca*, les extraits végétaux spécifiques riches en kampférol ou en kampférol glucosides et/ou riches en lutéine ou en autres flavonoïdes.

12. Capsule selon l'une quelconque des revendications précédentes, **caractérisée en ce que** les acides aminés sont choisis parmi la lysine, l'arginine et la taurine.

13. Capsule selon l'une quelconque des revendications précédentes, **caractérisée en ce que** les acides gras insaturés représentent des acides gras ω.

14. Procédé de préparation de capsules selon l'une quelconque des revendications 1 à 13, comprenant
- la sélection des principes actifs en fonction de leurs lieux de résorption ;
- la préparation d'au moins trois groupes de pellets de principes actifs ayant des profils de libération différents pour les lieux respectifs de résorption avec introduction des vitamines et éventuellement d'autres principes actifs qui sont répartis entre les groupes de pellets de principes actifs en fonction des profils de libération et des lieux de résorption et
- introduction des pellets de principes actifs dans une capsule.
